# EUROPEAN PATENT APPLICATION

(11) **EP 2 743 248 A1**
(43) Date of publication of application: **18.06.2014**
(21) Application number: 12811374.3
(22) Date of filing: 03.07.2012
(51) Int. Cl.: C07C 37/82, A23L 1/30, A61K 8/97, A61K 36/63

(54) **METHOD FOR OBTAINING HYDROXYTYROSOL EXTRACT, MIXTURE OF HYDROXYTYROSOL AND 3,4-DIHYDROXYPHENYLGLYCOL EXTRACT, AND HYDROXYTYROSYL ACETATE EXTRACT, FROM BY-PRODUCTS OF THE OLIVE TREE, AND THE PURIFICATION THEREOF**

(30) Priority: 08.07.2011 ES 201131161 P
(71) Applicant: Consejo Superior De Investigaciones Científicas (CSIC), 28006 Madrid (ES); Universidad De Sevilla, 41013 Sevilla (ES)
(72) Inventor: FERNÁNDEZ-BOLAÑOS GUZMÁN, Juan, 41012 Sevilla (ES); RODRÍGUEZ GUTIÉRREZ, Guillermo, 41012 Sevilla (ES); LAMA MUÑOZ, Antonio, 41012 Sevilla (ES); SENENT RUBIO, Fátima, 41012 Sevilla (ES); FERNÁNDEZ-BOLAÑOS GUZMÁN, José María, 41013 Sevilla (ES); MAYA CASTILLA, Inés, 41013 Sevilla (ES); LÓPEZ LÓPEZ, Óscar, 41013 Sevilla (ES); MARSET CASTRO, Azucena, 41013 Sevilla (ES)
(74) Representative: Ungria López, Javier
(86) International application number: PCT/ES2012/070491
(87) International publication number: WO 2013/007850

(57) **Abstract**

The invention relates to a method for obtaining an extract comprising hydroxytyrosol, as an acetylated product or as a mixture with 3, 4-dihydroxyphenylglycol and the purification thereof, comprising at least one column chromatography of a hydroxytyrosol source such as by-products of the olive and the olive tree, using a mixture of at least two ionic resins, preferably an anionic resin with a cationic resin. Preferably, in a first phase, a phenyl-rich liquid is obtained as the raw material for the extraction and purification of phenolic compounds, a second phase enables a hydroxytyrosol (HT)-enriched extract and hydroxytyrosol with 3, 4 dihydroxyphenylglycol (DHFG) mixture to be obtained, and hydroxytyrosol acetate is produced, and, in the third phase, highly pure hydroxytyrosol, DHFG and the mixture thereof and HT acetate are obtained. Each phase comprises extraction, reaction, concentration, adsorption and desorption, using mixtures of ion exchange resins, adsorption in non-ionic resins and a polymer phenolic fraction, membranes of reverse osmosis and evaporators.

## Description

### TECHNICAL FIELD

The art object of the present patent is directed towards a broad industrial sector that not only encompasses agriculture but also the cosmetic, food, nutraceutical and pharmacological sectors. In general, its use shall be determined by the properties largely derived from the natural antioxidant potential of hydroxytyrosol (HT), its acetate and its mixture with 3,4-dihydroxyphenylglycol (DHFG) and its high biological activity for preventing, within the formulation of functional foods or drugs for cardiovascular or neurodegenerative diseases, cancer or other conditions related to the anti-inflammatory, antimicrobial, etc. activity thereof.

### STATE OF THE ART

Epidemiological studies indicate that the Mediterranean diet is associated with a lower incidence of cardiovascular disease, atherosclerosis and certain types of cancer (skin, breast, prostate and colon) (López-Miranda et al., 2010). It is a diet rich in fresh fruits and vegetables, with a relatively low proportion of animal fat, olive oil being the main source of fat. These beneficial effects have not only been attributed to a low proportion of saturated / monounsaturated fatty acids of olive oil but also to additional molecules present in lower concentrations, in particular, antioxidant phenolic compounds.

Nowadays, interest in natural antioxidants is increasing because growing evidence indicates that they are able to counteract the nitrogen and cytotoxic effects of free radicals and reactive oxygen species, which occur in cases of oxidative stress and are involved in several pathological processes, such as kidney and liver diseases, inflammatory processes and cancer (Menéndez et al., 2008). For this reason, several of them are isolated from olives, hydroxytyrosol (3, 4-dihydroxyphenylethanol) being highlighted amongst all of them.

According to the "European Olive Oil Medical Information Library", hydroxytyrosol (HT) is a phenolic compound of an antioxidant particularly renowned for efficacy. Its antioxidative properties are particularly attributed to the presence of two ortho position hydroxyl groups, i.e., an orthodiphenol group, characteristic of biophenols. In addition to its ability to donate electrons and neutralize free radicals, its high antioxidant efficacy lies in its ability to sequestrate or chelate metal Fe or Cu ions, among others, responsible for the formation of free radicals during redox processes.

In recent years, it has been found that this natural HT antioxidant also has important biological properties. Evidence points towards its neuroprotective potential, protecting mice brain cells. It also presents a range of functions that are related to cardiovascular protection, such as stimulation of the defense, detoxification and antioxidant transcription system, alteration of the expression of genes related to the development and progression of atherosclerosis and / or cardiovascular protection, improvement of lipid profile, increase of antithrombotic effects and anti-inflammatory markers decrease (cyclooxygenase-2, thromboxene, C reactive proteins, etc.). Furthermore, the ability of these compounds to remain bound to LDL proteins, protecting them from oxidation, has been demonstrated. It has also been demonstrated that HT induces a wide range of antitumor effects, including suppression of proliferation and apoptosis induction in colon carcinoma cells.

Currently, there is, therefore, much research attempting to demonstrate the action of this compound in the prevention and treatment of many diseases. It prevents macular degeneration, ischemic cerebral strokes and aging skin and improves vision. All this background also suggests the use of HT as a functional component in food, as well as its use for preventing deterioration of the same owing to its proven ability to inhibit the oxidation of unsaturated lipids.

Various methods and systems directed towards obtaining extracts rich in HT, in addition to those which try to isolate and purify the HT component from olives and the by-products thereof, as well as from the olive tree leaves, have been developed and patented.

In many cases solvent extraction techniques or methods based on selective separation and concentration by ultrafiltration, reverse osmosis, evaporation, chromatographic separation and extraction with supercritical fluids are used, from both vegetable waters separated from olive waste ("alperujo"), as well as waters for processing table olives. These techniques may be used individually or in an integrated way. Therefore, after carrying out an ultrafiltration of the water generated in the processing of table olives, adsorption in non-ionic resin (XAD type) was carried out, so as to obtain an extract rich in hydroxytyrosol (ES 2.186.467). Microfiltration, ultrafiltration, nanofiltration and reverse osmosis membrane separation techniques, followed by a column purification process (U.S. 2006/0070953) have also been described.

Moreover, hydroxytyrosol extracts are also obtained from olive vegetable waters by means of supercritical fluids, such as carbon dioxide (US 2002/0198415).

One of the most simple, practical and economic procedures of those described is that which allows a hydroxytyrosol with a high degree of purity to be obtained from any aqueous source (alpechin, aqueous phase of the hydrothermal "alperujo" process, washing water from the elaboration of table olives, etc.) by means of chromatography in two steps, namely a first step with an ion exchange resin and a second one with an adsorbing resin of the XAD type (US 2004/0102657).

HT extraction from alpechin (olive waste water) by means of a mixture of ethyl acetate and ethanol or ethyl acetate, ethanol and water, so as to subsequently carry out treatments of the organic phase with an XAD resin type, which allows extracts with high concentrations of HT to be obtained (ES 2311401) has also been described.

Among the existing synthesis methods, the one described by Capasso et al. (Capasso, R., Evidente, A., Avolio, S., and Solla, F. 1999. J. Agric. Food Chem. 47: 1745-1748), is, according to the author himself, the most suitable compared to the others described in the literature (Balardi, P. G., Simoni, D., Manfredini, S. and Menziani, E. 1983. Liebigs. Ann. Chem. 684-689). Hydroxytyrosol is synthesized from 3, 4-dihydroxyphenylacetic acid by reduction with LiAlH₄ in tetrahydrofuran, under reflux, for two hours.

Bai et al. (Bai, C., Yan, X., Takenaka, M., Sekiya, K. and Nagata, T. 1998. J. Agric. Food Chem. 46: 3998-4001), also synthesized it with a high yield, on a gram scale and with high purity. Synthesis is carried out based on the same 3, 4-dihidroxypenylacetic acid but using a methylation system with trimethylsilyldiazomethane and subsequent reduction of the ester obtained with NaBH₄. More recently, it is obtained by reducing the phenylacetic acid methyl ester with sodium borohydride (WO 2007/009590) or by oxidation of tyrosol in the presence of hydrogen peroxide and methyl rhenium trioxide (US 2009/0023815).

It is obtained enzymatically from tyrosol using a mushroom tyrosinase (ES 2170006) as a catalyst.

In addition to HT, its monoacetylated derivative, hydroxytyrosyl acetate is also a natural compound that has been detected in virgin olive oil. This is a somewhat lipohilic compound with an antioxidant effect similar to HT, therefore meaning it would serve many applications as a functional additive in fatty foods. A method for obtaining hydroxytyrosyl monoesters from HT by means of a regioselective esterification reaction on the aliphatic hydroxyl (ES1541544) has been described. It has been demonstrated that this compound exerts a greater anti platelet aggregation effect in humans than HT, similar to the effect exercised by acetylsalicylic acid (González Correa et al. 2009). A process for producing triacetylhydroxytyrosol by means of an acylating mixture of perchloric acid and acetic anhydride that makes it more chemically stable than HT and more readily miscible with lipophilic, pharmaceutical and cosmetic preparations (WO 2007/074490) has also been described.

Similar to HT and somewhat less studied, is 3, 4-dihydroxyphenylglycol (DHFG), the structure of which is like that of HT with an additional hydroxyl group in the benzylic position. Its antioxidant efficacy in water is two or three times higher than that of ascorbic acid or HT, whilst in a lipidic medium, it is double that of HT and similar to vitamin E in *in vitro* assays (Rodriguez et al., 2007).

It has now been demonstrated that it has a significant synergistic effect with HT to inhibit platelet aggregation, the mixture of which moreover equals the effect exerted by all the phenols present in an extract containing the major alperujo phenolic compounds (Roos et al., 2011). This new phenol is not currently on the market, the obtainment thereof being a significant advance owing to its bioactive potential, mainly mixed with HT, in order to take advantage of the potential synergistic properties thereof.

### DESCRIPTION OF THE INVENTION

The present invention refers to a method for obtaining an extract comprising hydroxytyrosol, either as such, as an acetylated product or as a mixture with DHFG and the subsequent purification of all of them characterised in that this method comprises carrying out at least one column chromatography, a source of hydroxytyrosol, using a mixture of at least two ionic resins, preferably an anionic resin and a cationic resin.

In the event that a liquid source of the compounds is not sought, or if it is important to increase the concentration thereof, a previous preparation of the hydroxytyrosol source is carried out, obtaining a liquid source with a high phenol content, mainly in hydroxytyrosol and DHFG treated before being passed through the column. The source preparation has to provide a liquid low in suspended solids and fats that allows the percolation thereof by means of the resins also extending the lifespan of the resins. Sources lacking DHFG may also be used, in this case obtaining a HT extract, another extract from the acetate thereof and these purified compounds.

According to the above, if the hydroxytyrosol source is solid or semisolid, the source preparation comprises an extraction in liquid phase and separation of the liquid phase by means of filtration, centrifugation and/or decantation.

Optionally, if the source of hydroxytyrosol is liquid, the preparation thereof comprises carrying out a hydrolysis process.

The sources of hydroxytyrosol may be by-products of olives and the olive tree, for instance alpechin, olive pomace, "alperujo", leaves, pruning, bone, seasoning process water, Oleaceae family plants and combinations thereof.

According to the method object of the invention, the resins may be cationic, anionic or combinations of both types. Preferably, according to the invention, a mixture of anionic with cationic resin is used, and more preferably still, said mixture comprises at least 2% by weight of the total mixture in cationic resin. Any percentage higher than 2% by weight produces an optimal result. The use of at least one cationic resin favors adsorption of simple phenols and extends the life of the resin.

The following, amongst others, may be used as useful resins for putting the method object of the invention into practice:

Cationic resins, including the entire series of Imac HP, the entire series of Amberlite FPC, Amberlite IRP, Amberlite CR, Amberlite IR, Amberlite SR, Amberlite IRC, Amberlite PWC; the entire series of Amberjet 1000, 1200, 1500, 4200, 4600 or 252; the entire series of Dowex, Dowex Monosphere, Dowex HCR, cationic Dowex SBR; resins of the variety from the Lewatit monoplus, IRA or S series, cationic Ambersep or Amberlyst resins.

Anionic resins, including the entire series of Amberlite, Amberlite IRA, Amberlite FPA; the entire series of Amberjet 4200, 4400 or 4600; the entire series of Dowex, Dowex Monosphere, Dowex HCR, anionic Dowex SBR; Lewatit type resins, anionic Ambersep or Amberlyst and the entire series of Duolite A, AP resins.

In the event of carrying out a partial activation of the resin mixture, said activation may be carried out with acidic, alkaline or saline solutions. Said resin mixture may be heated to temperatures of at least 50°C during regeneration.

According to the method object of the invention, in order to carry out the chromatography, the hydroxytyrosol source is passed through the column and is emptied, or not, once it has finished said loading of the source. After loading the source on the column, the source, which continues to impregnate the resin, may optionally be emptied. If the source is emptied, the transport thereof is better facilitated and the first washes will furthermore be obtained with a higher degree of purity in the compounds sought, mainly DHFG, the more polar nature of which elutes in the first washes.

According to particular embodiments of the method for carrying out the column, the hydroxytyrosol source is loaded in the location where the same source is generated by moving columns or in plant.

According to the invention method, the elution of the extract obtained from the column is carried out, according to preferred embodiments, in sequence: firstly with neutral or acidified water in a first series of elutions in a first discharge step, again with neutral or acidified water in a second series of elutions in a second discharge step, acidified water with a pH of between 2 and 6.5 preferably being used in both series.

Alternatively, the sequential discharge of the column may comprise a HT acetylation reaction by means of a reaction phase in the column in which the HT is adsorbed, which, after carrying out the second series of elutions, comprises:
- emptying the column,
- optionally drying by introducing hot air,
- subsequently completely flooding the column with ethyl acetate, closing the inlet and outlet of the column and heating it preferably above 50°C,
- and emptying the column once again, which may be accompanied by a final elution step with more ethyl acetate to completely elute the hydroxytyrosyl acetate formed.

This embodiment may also comprise evaporating and crystalizing the hydroxytyrosyl acetate in an aqueous/organic or aqueous medium.

According to a second alternative for the acetylation of HT, this comprises:
- Carrying out a column chromatography, obtaining hydroxytyrosol extract or already purified hydroxytyrosol, putting the cationic resin in contact with said hydroxytyrosol extract or purified hydroxytyrosol and carrying out an acetylation reaction in a stirred tank, applying a temperature above 50°C in presence of ethyl acetate. This embodiment may also comprise evaporating and crystalizing the hydroxytyrosyl acetate in an aqueous/organic or aqueous medium.

According to the invention method, the extract obtained may be purified according to one or more of the following operations:
- carrying out an absorption chromatography with non-ionic resins and subsequently desorption with neutral or acidified water or the mixture thereof with organic solvents.
- purification by adsorption on a new polymeric phenolic fraction (PPF) obtained from the products and by-products of the olive oil industry, this fraction retaining the extract formed by hydroxytyrosol and 3,4-dihydroxyphenylglycol, and redissolution of said hydroxytyrosol and 3,4-hydroxyphenylglycol in an aqueous or organic medium at room temperature or above, together, obtaining the purest mixture, or differential mixture, obtaining on the one hand HT and on the other hand DHFG, both with a higher degree of purity by weight. Hydroxytyrosol extract, which is redissolved in water to obtain a HT with a purity of up to 100%, may be retained in said fraction.
- concentration of the purified extracts by means of evaporation, atomization or reverse osmosis concentration, thus obtaining dry or partially wet products.

Therefore, DHFG may be obtained by putting the HT + DHFG mixture in contact with the PPF and washing, or performing an extraction with water at room temperature, obtaining HT in a first wash, HT+DHFG in a second wash and DHFG in the third, all of them having a higher purity.

The present invention also has a product selected from the hydroxytyrosol, hydroxytyrosyl acetate and 3,4-dihydroxyphenylglycol compounds, and combinations of said compounds, as the object thereof, it being possible to obtain said product by means of the process described.

One preferred product from the method is a mixture of hydroxytyrosol and 3, 4-dihydroxyphenylglycol in a minimum ratio of 10:1 and a maximum ratio of 1:2.

The present invention also has a mixture of hydroxytyrosol and 3, 4-dihydroxyphenylglycol in a minimum ratio of 10:1 and maximum ratio of 1:2, and preferably in a minimum ratio of 5:1 and maximum ratio of 1:1.3.

A product obtained according to the invention method is selected from:
- Hydroxytyrosol with a purity between 80-100%,
- A mixture of hydroxytyrosol and 3, 4-dihydroxyphenylglycol with a purity between 60-100% as a sum of the individual units,
- 3, 4-dihydroxyphenylglycol with a purity between 50-100%, and
- Hydroxytyrosyl acetate with a purity between 80-100%.

The present invention also has a phenolic extract as the object thereof, characterized in that it comprises one or more of the products defined above.

Said extract may preferably be obtained by means of the process described.

The present invention also has an additional object constituting the use of the extract defined above or the product defined above as an antioxidant, anti-inflammatory, antimicrobial, cell antidegenerative, anticancer and free radical sequestration agent in food, cosmetic and pharmaceutical formulations.

The present invention also has an additional object constituting the use of the extract defined above or the product defined above as an agent taking part in emulsions, nano and microencapsulations or adsorbed in other substances to improve the physico-chemical properties and/or stability thereof.

Taking into account that hydroxytyrosol and DHFG are both antioxidants, DHFG being more powerful than HT yet more unstable, the presence of HT stabilizes it. This combination of properties gives rise to a synergic effect on the mixture of hydroxytyrosol and DHFG, which strengthens the activity of each one separately. Therefore, the combined use of hydroxytyrosol and DHFG, i.e. as a mixture, in food, cosmetic and pharmaceutical formulations is particularly advantageous.

Likewise, the mixture of HT with hydroxytyrosyl acetate enables the use of two antioxidants with different polarities, which could be very effective for preventing oxidative states in mixtures of hydrophilic and lipophilic media, which are or are not forming emulsions in foods, cosmetic products or drugs.

Therefore, the present invention has as an additional object constituting the combined use of, on the one hand, hydroxytyrosol and DHFG and on the other hand, HT and hydroxytyrosyl acetate in mixtures of hydrophilic and lipophilic medium, which are or are not forming emulsions in a formulation selected from the formulation of foods, cosmetic products or drugs.

The basic method of the present invention is applicable to those products and by-products derived from olives and olive trees containing hydroxytyrosol and therefore susceptible for use as a source of this natural antioxidant.

### DETAILED DESCRIPTION OF THE INVENTION

In a particular embodiment of the invention, the method may comprise three phases as schematically indicated in the diagram below:

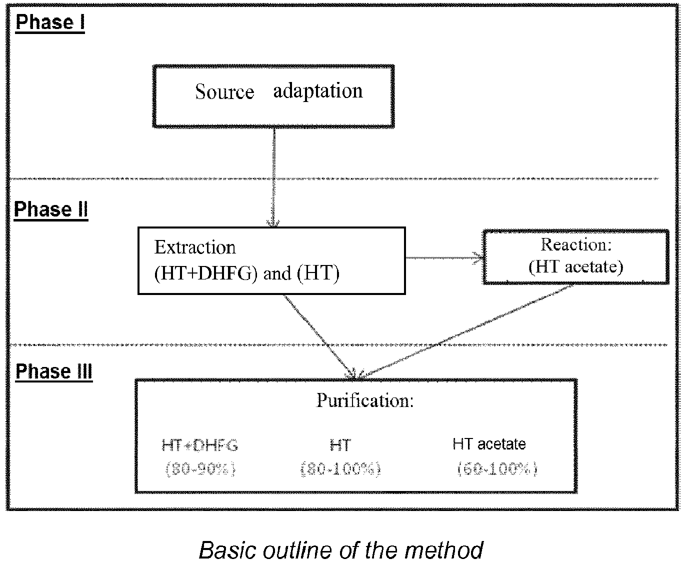

The phases of the method according to this embodiment are as follows:
1. Source preparation, which is optional, as in some cases a liquid source with hydroxytyrosol is available and there is no need to pretreat it, as is the case with alpechines which have been stored for a long time or dressing waters with a high hydroxytyrosol content.
   In the event of not having a liquid source of HT and DHFG, or if they are present in a low concentration in said source, the source is prepared to obtain a liquid solution containing free HT and DHFG in high concentrations. If there is a liquid source available, which can pass through the resin, that phase would therefore be optional in order to increase the concentration thereof.
2. Obtaining three extracts: a HT extract, a second extract comprising a mixture of HT with DHFG and a third extract rich in HT acetate.
3. Purification of each one of the extracts, obtaining HT, HT acetate, DHFG and a mixture of HT and DHFG, all of them with a high degree of purity by weight.

Each phase enables marketable products to be obtained, as they are a source of phenols, extracts and compounds or highly purified mixtures.
Each one of the phases that the method may comprise, according to this embodiment, is in turn divided into several steps, which are detailed in Diagram 1 and explained below:

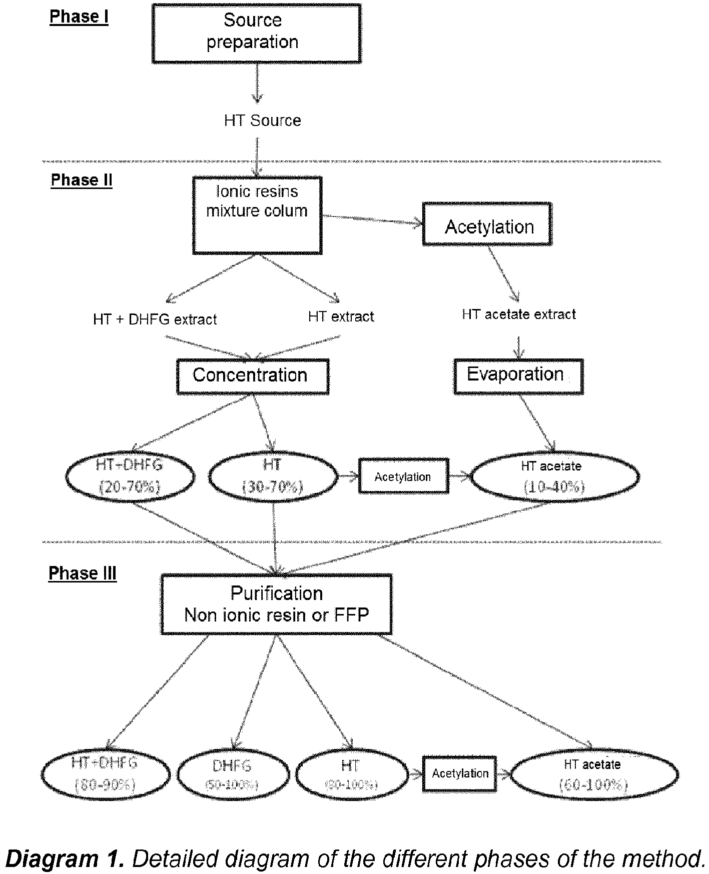

### PHASE I

In this phase, the hydroxytyrosol source is prepared until a liquid fraction containing HT and/or DHFG is obtained. To do this:
- In the event of the hydroxytyrosol source being solid or semisolid (olive pomace, "orujillo", olive leaves, pruning or "alperujo") a liquid extraction phase with water in acidic or alkaline medium, or with an organic solvent such as ethanol, or with a mixture of both, is carried out. The extraction conditions may be preceded by grinding to maximize the contact surface. The extraction may be enhanced by applying heat. The different phases would finally be separated by means of centrifugation, filtration and/or decantation, obtaining a liquid with low or no suspended solids and/or fatty matter content.
- In the event that the source is liquid whereas the HT and/or DHFG content thereof is very low in a free form, nevertheless having precursors such as oleuropein, 2"hydroxyoleuropein, verbascosyde or β-hydroxyverbascosyde among others, a previous hydrolysis would be carried out, catalyzed or not with an acid or an alkali, which would hydrolyze said compounds, releasing HT and/or DHFG into the environment. Subsequently, a pre-sedimentation or centrifugation may or may not be carried out, so as to eliminate the potential presence of suspended solids and/or fats, which may be enhanced by the addition of acids, alkalis and/or flocculants.

### PHASE II

In the first phase, the raw material for the extraction and purification of phenols or phenolic extracts obtained in the second phase is obtained, as are HT, hydroxytyrosyl acetate and the mixture of HT and DHFG. In this phase II, an extract of hydroxytyrosol (HT) rich in said antioxidant and in other simple phenols, a bioactive HT and 3, 4-dihydroxyfenylglycol (DHFG) are obtained, in addition to enabling the corresponding hydroxytyrosyl acetate to be formed.

The source obtained in phase I or the liquid source obtained directly, be it an aqueous, organic or a mixture of both media, is subjected to a chromatographic column. The resin used is a mixture of anionic exchange resin with at least 2% cationic. This provides a number of significant advantages in the process:
- Greater retention specificity in simple phenols is achieved and it favors the washing and regeneration steps, reducing the effluents produced in the regeneration phases.
- The resins mixture along, optionally, with the use of water with an acidic pH as an eluent, enables a HT with a higher purity degree and a more stable mixture of HT with DHFG, to be obtained.
- This resin mixture makes the further acetylation step in which the acid resin acts as a catalyst possible, thus preventing the use of enzymes and/or acids.
- The extract is further enriched in hydroxytyrosol and analogous phenols, such as DHFG, thus enabling an extract rich in both phenols, which present synergistic activities, to be obtained.
- The new resin mixture is poisoned or absorbs fewer organic compounds, mainly of a lipidic origin, substantially extending its life and reducing the regeneration cycles and the effluents produced from it.
- Activation is simplified to a partial activation with saline solutions, which may be used in more than one cycle and the action of which is favored by means of temperature, preventing frequent regenerations with strong acids or alkalis and thereby, the effluents thereof.
- The new resin mixture improves the option of being able to empty and evacuate the liquid contained in the column and, as a result, impregnating the resin. This has three important advantages, amongst others:
- It prevents the eluents from mixing with the source left inside the column at the beginning of the discharge or elution of the column, also enabling more purified extracts to be obtained from the first elutions.
- It also facilitates being able to load the source "in situ", i.e. in the same place as where it is originated, without needing to transport the entire source or the fraction which remains in the column, to the place where the next steps would take place, operating by way of mobile columns. This prevents the source from being handled once it has passed through the column, since it would be a pollutant effluent to treat, also reducing transport costs substantially, limiting them to the transport of the columns themselves, without liquid inside.
- An improved emptying facilitates the subsequent hydroxytyrosyl acetate production phase, as it is important to eliminate as much water as possible, the presence in the ethyl acetate and HT reaction media of which originates secondary reactions which compete with the production of HT acetate.

The way to operate with the column comprises several steps:
1. A first partial activation with saline solution, which may be favored by the temperature through a heating jacket, followed by washing with water or slightly acidified water, even up to a pH of 2. When the resin mixture has been subjected to many cycles of use, heating over 50°C would be jointly applied, in order to regenerate the same or even a previous washing with acid and/or alkali.
2. Source loading over the resin may be carried out by means of gravity or flooding of the bed, inside a processing plant or even in the industry where said liquid source is produced. Carrying out loading in the same industry where the source is produced would have the advantage of avoiding source effluents once passed through the column. For the industry, it would have the advantage of obtaining a liquor with a lower organic load, diminishing DBO, DQO and the content in phytotoxic compounds which may hinder the purification thereof. After loading, a total evacuation of the source may be carried out, leaving the resin as dry as possible. This results in first washings or elutions with water not dragging the original source and the DHFG and HT collected being eluted more easily with water and a higher degree of purity.
3. Column discharge may be carried out in three steps, obtaining a different extract in each one of them, it being possible to use slightly acidified water in the first two to facilitate the ionization of compounds and thereby, the better elution thereof and better conservation of the product obtained:
   3.1. In a first series of elutions with water, a mixture of HT and DHFG in a ratio from 10:1 up to 1:2 of both respectively is obtained.
   3.2. In a second series of elutions with water an extract rich in HT is collected, the average purity of which is between 30-70% by dry weight thanks to the new resin mixture and the elution with water at an acid pH.
   3.3. After the second series of elutions with water, the column is emptied and hot air is optionally introduced so as to remove as much water remaining in the column as possible. The column is subsequently flooded completely with ethyl acetate, closing its inlet and outlet of the same and is heated with a heating jacket or an electrical heating coil inside the bed, so that the HT which is still retained in the column is acetylated due to the catalytic action of the resin mixture. Finally, it is emptied or even more ethyl acetate may be added for the total elution of the column obtaining an extract rich in HT acetate with an average purity of 10-40% by dry weight.

The three extracts obtained may be concentrated to even dryness using reverse osmosis equipment, evaporation equipment with or without vacuum or another concentration system. Final extracts with a high biological activity are thus obtained. In the event of the new mixture of HT with DHFG, the interest lies in its marked synergic character, fostering certain activities, as is the case with the inhibition of platelet aggregation, in addition to obtaining a more stable and easily manageable final product in this way.

### PHASE III

Once the extracts are obtained, the same may be purified in order to obtain a HT and the acetate thereof with a high degree of purity, both in the range of 80-100%. In the case of the HT-DHFG mixture its purity increases up to 60-90% and its components may even be separated by adsorption in a new polymeric phenolic fraction (PPF) developed by us. This fraction has been disclosed in patent application number P201131041. This mixture consisting of the union of condensed phenols with an undetermined structure halves them to be obtained individually in a pure state or in a purity range of 80-100% for HT or 50-100% for DHFG. PPF is obtained from olive by-products (alpechin, olive pomace, "alperujo", pruning, or by-products of the dressing industry) thermally treated at above 50°C for 30 to 180 minutes and later extracted with ethyl acetate. The PPF forms part of said extract and may be purified to a greater or lesser extent by using non ionic resins or by gel filtration among others. This polymeric fraction presents a curve in the HPLC profile at 254 and 280 nm wavelengths, characteristic in a complex structural framework that does not allow the separation of its components. It is identified by means of elution on C-18 HPLC with an acetonitrile-water gradient where it elutes in the range of 20% acetonitrile and 80% water to 40% acetonitrile and 60% water.

Purification may proceed in different ways, in order to reach an adsorption process:
1.-Purification in adsorption columns: The HT extract, the acetate thereof or the HT-DHFG mixture obtained in the second phase are passed separately through a non-ionic or adsorption column of the XAD type, from which they are eluted with an ethanol-water gradient or simply with water, in either case the aqueous fraction may be slightly acidified (pH 2-6.5) so as to facilitate the ionization of the species and improve the desorption performance of the DHFG, HT and the acetate thereof. HT and a HT acetate with a purity comprised between 80-100% is thus obtained in both cases. HT acetate may be dissolved in a mixture of organic solvent with water and the organic solvent subsequently removed, until the acetate crystalizes, so as to increase the degree of purity thereof.
   The HT acetate may alternatively be obtained from HT that has already been purified during phase III or even from the extract obtained during phase II. In either case, the HT is loaded in a stirred tank in the presence of an acid or cationic exchange resin and is heated until acetylation takes place. Therefore, when beginning with HT that has already been purified, a purer acetate is obtained.
2.-Purification by adsorption on a new polymeric phenolic fraction (PPF) obtained by solvent extraction from by-products of the olive. PPF has recently been discovered, as well as its capacity to retain simple phenols such as those treated in the present invention. Once obtained, the extracts are put into contact with the PPF in an aqueous, organic or a mixture of both media. The mode of operation depends on the solvents used:
   - Use of organic solvents: in this case, PPF is used dissolved in organic solvents mixed or not with water. This is the case of ethanol-water wherein PPF is redissolved and the extract to be purified is added to it, be it HT, DHFG or a mixture of both. Finally, the solvent is evaporated and water is added to it, with or without heating or stirring, obtaining a final dissolution with a high degree of purity in the phenols sought.
   - Use of Aqueous dissolutions: in this case, contact is favored by heating and stirring after adding the extract to the PPF. After heating, the aqueous phase may be immediately removed and water may be added for desorption of the compounds with high purity. This kind of contact may be carried out in a stirred tank or in a column where PPF is attached to a solid support forming part of the column packing. In this way, the column is initially flooded with the dissolution to be purified and the bed may or may not be heated. Finally, the column is evacuated and reloaded with water for the final desorption of the desired phenols: the HT, DHFG and their mixture with a higher degree of purity by weight.

### EMBODIMENT OF THE INVENTION

Hereinafter, by way of an illustrative and non-limiting example, the obtaining at a pilot-plant scale of HT, HT acetate and a mixture of HT with DHFG, is shown, according to a three step process:

### Phase I

It starts from a liquid source of alpechin, the concentration of which does not exceed 0.8 g/L in HT and 0.3 g/L in DHFG and a large amount of suspended solids. The source is acidified to a pH of 2.8 with phosphoric acid and left in a decanter to assist the precipitation of the fines for conditioning. The acid helps on the one hand hydrolysis of HT conjugates in order to increase the content thereof in a free form and on the other hand the separation of the solid. The supernatant is centrifuged, thus obtaining 350 L of source with a HT concentration of 1.6 g/L and a DHFG concentration of 0.3 g/L respectively.

### Phase II:

The already conditioned source (350 L) is passed through a column of 40 cm in diameter with a bed height of 115 cm, filled with a mixture of anion A-4200C type resin with cationic IR-120H type resin at 20%. The mixture has previously been partially activated with a saturated saline solution (NaCl) and washed with water acidified to a pH of 2. The column also has a heating jacket and a hot air inlet. Once the source has been evacuated from the column, the various fractions thereof are eluted and treated.
- First elution: 550 L of acidified water (pH 2-6) are used so that the solution at the outlet has a pH of between 6.5 and 7. An extract rich in HT with DHFG mixture is obtained, in a ratio of about 4:3. This extract is concentrated in a piece of reverse osmosis equipment until 5L of final extract **(extract M)** is obtained at a concentration of 22.6 g of HT/L and 14.6 g of DHFG/L, the total purity of the mixture being 30% by weight.
- Second elution: the process continues by eluting with 300 L of acidic water; in this case the output pH is not controlled since DHFG is no longer sought. Subsequently, the elution is concentrated in the reverse osmosis equipment until 2 L of **HT extract** with 90 g of HT/L and a purity of 63% by dry weight is obtained.
- In a third step, the column is dried by evacuating all the water and passing a stream of hot air through it. The dry column has adsorbed about 260 g of HT. The bed is flooded with ethyl acetate and the column is heated at 70°C with the heating jacket. After a period of two to three hours, the column is evacuated once again and the ethyl acetate rich in HT acetate is concentrated, obtaining 440 g of an extract **(HT Acetate extract)** at 18% of HT acetate (about 80 g).

### Phase III.

The extracts obtained are purified by applying two adsorption methods; on the one hand by means of the non-ionic adsorption columns and on the other, by means of a new polymeric complex called PPF.
A) Adsorption in resins: one half of each one of the three extracts (HT with DHFG mixture, HT, and HT acetate) is purified by means of a column of 10 cm in diameter and 70 cm height with non-ionic adsorption EXA or XAD type resin.
   - **Extract M:** the column is loaded and the HT, along with the DHFG, is eluted with 20 L of a gradient of 20-40% of ethanol in water. The purified mixture is concentrated in the reverse osmosis equipment up to 1 L obtaining a mixture of 127 g which contains 30 g of DHFG and 47 g of HT with a final purity of above 60% by weight.
   - **Extract HT:** the column is loaded and eluted with 40 L of acid solution, obtaining 89 g of an extract containing 82 g of HT with an average purity of 92%. Elution may also be carried out using an ethanol-acid water gradient of up to 35% together with the acid water, thus obtaining less elution volume.
   - **Extract HT acetate:** is redissolved in ethanol/water (1:1) and is passed through the column and eluted with an ethanol-water gradient from 10% up to 80%, obtaining a 12 L fraction with 2.7 g/L of HT acetate, which contains 85% of that loaded in the column. The 12 L are evaporated and 35.8 g are obtained containing 35 g of HT acetate with a purity of 97.8% by dry weight. HT acetate is also obtained starting with already purified HT. To do this, 41.7 g of purified HT containing 40 g of dry HT with a purity of above 96% are mixed with 150 g of cationic IR-120H type resin and 0.5 L of ethyl acetate in a closed recipient, with stirring and heat under reflux. After 11 hours 74% of HT would be acetylated therefore obtaining 37.7 g of HT acetate.
B) Adsorption on PPF: The other half of each one of the extracts is purified by means of adsorption in the new polymeric phenolic fraction.
   - **Extract M.** Starting with 152 g of extract containing 56.5 g of HT and 36.5 g of DHFG in 0.5 L of aqueous solution, to which 600 g of PPF and 0.5 L of ethanol are added. The mixture is stirred and evaporated to dryness. It is finally redissolved in 0.5 L of water and the insoluble PPF is separated, thus obtaining an aqueous fraction with 50.8 g of extract containing 20 g of HT and 13 g of DHFG with a purity of 65%. It is washed once again with 0.5 L of water and 65.5 g of extract containing a mixture of 35 g of HT and 22 g of DHFG with a purity of 87% by weight.
      Similarly, another test starting with 50 g of another extract obtained in another experiment containing 16.3 g of HT and 14.5 g of DHFG with a mixture purity of 61.6% by dry weight is carried out. 200 g of the PPF and 0.25 L of ethanol are added. It is stirred and evaporated to dryness. This time it is redissolved in several washes with cold water, obtaining 7.8 g of HT with a purity of 93% in the first wash, a mixture of 8.2 g of HT and 6.7 g of DHFG with a purity of the mixture of 82% in the second wash and finally 4.3 g of DHFG with a purity higher than 70% by dry weight in the third wash.
   - **HT Extract:** starting with 90 g/L of existing HT with a purity of 63% in 1 L, to which 600 g of PPF and 0.5 L of ethanol are added. It is taken to dryness and redissolved in 1 L of water and the insoluble PPF is separated, thus obtaining a 45 g extract which contains 36 g of HT with a purity of 80%. The PPF is once again washed with another liter of water and 52 g of totally pure HT is obtained.

## Claims

1. Method for sequentially obtaining extracts with a high concentration of hydroxytyrosol, in a mixture of hydroxytyrosol and 3, 4-dihydroxyphenylglycol and in hydroxytyrosol acetate, **characterized in that** said method comprises carrying out at least one column chromatography of a hydroxytyrosol source, using a mixture of at least two ionic resins, preferably an anionic resin with a cationic resin.

2. Method according to claim 1, **characterized in that** the hydroxytyrosol source is solid or semisolid and one previous step a) is carried out, which comprises carrying out a liquid phase extraction and separation by filtration, centrifugation, decantation or a combination of centrifugation and decantation.

3. Method according to claim 1, **characterized in that** the hydroxytyrosol source is liquid and a previous step a) is carried out, which comprises carrying out a hydrolysis.

4. Method according to claim 1, **characterized in that** the hydroxytyrosol source is selected from alpechin, olive pomace, *alperujo,* leaves, pruning, bone, dressing process waters, oleaceae family plants and combinations thereof.

5. Method according to claim 1, **characterized in that** the anionic resin and cationic resin mixture contains at least 2% by weight of the total mixture in cationic resin.

6. Method according to claim 1, **characterized in that** a partial activation of the resin mixture is carried out at room temperature with a solution selected from an acid solution, an alkaline solution and a saline disolution, or with a solution selected from an acid solution, an alkaline solution and a saline dissolution, heating at temperatures of at least 50°C during regeneration.

7. Method according to claim 1, **characterized in that** the hydroxytyrosol source is passed through the column and said source is emptied before passing an eluent in order to carry out the chromatography.

8. Method according to claims 1 or 7, **characterized in that**, in order to carry out the column chromatography, the hydroxytyrosol source is loaded in the same place where the source is produced by mobile columns or in plant.

9. Method according to claim 1, **characterized in that** it comprises a hydroxytyrosol acetylation step after the elution of the column.

10. Method according to claims 1 or 9, **characterized in that** the column chromatography comprises a sequential discharge of said column with neutral or acidified water in a first series of elutions in a first discharge step, and with neutral or acidified water in a second series of elutions in a second discharge step.

11. Method according to claim 9 or 10, **characterized in that** when carrying out the column chromatography with a mixture of ionic resins, the sequential discharge of the column comprises a third step:
- emptying the column,
- subsequently completely flooding of the column with ethyl acetate, closing the inlet and outlet of the column and heating,
- and emptying the column once again.

12. Method according to claim 1 or 10, **characterized in that** an acetylation is carried out, which comprises:
- carrying out the column chromatography obtaining hydroxytyrosol extract or already purified hydroxytyrosol, putting a cationic resin in contact with said hydroxytyrosol extract or purified hydroxytyrosol and carrying out the acetylation reaction in a stirred tank, applying a temperature of above 50°C in the presence of ethyl acetate.

13. Method according to claim 1, **characterized in that** it also comprises a purification step for the extract obtained, which comprises carrying out one or more of the following operations:
- carrying out an adsorption chromatography with non-ionic resins and subsequently desorption,
- purification by means of adsorption on a polymeric phenolic fraction, said fraction retaining hydroxytyrosol or the extract formed by hydroxytyrosol and 3,4-dihydroxyphenylglycol, and redissolution of said hydroxytyrosol and 3,4-dihydroxyphenylglycol in a joint or individual manner,
- concentration of the extract or the purified compounds by means of evaporation, atomization or reverse osmosis, obtaining a dry or partially wet product.

14. A product selected from the compounds hydroxytyrosol, hydroxytyrosol acetate and 3, 4-dihydroxyphenylglycol, and combinations of said compounds, **characterized in that** said compound may be obtained by means of the method described in one of the claims 1 to 21.

15. A product which is a mixture of hydroxytyrosol and 3, 4-dihydroxyphenylglycol in a minimum ratio of 10:1 and a maximum ratio of 1:2.

16. A product according to claim 14, **characterized in that** it is selected from:
- hydroxytyrosol with a purity between 80-100%,
- a hydroxytyrosol and 3, 4-dihydroxyphenylglycol mixture with a purity between 60-100% as a sum of the individual ones,
- 3, 4-dihydroxyphenylglycol with a purity between 50-100%, and
- hydroxytyrosyl acetate with a purity between 80-100%.

17. A phenolic extract **characterized in that** it comprises the product defined in one of claims 14 to 16.

18. A phenolic extract **characterized in that** it is obtainable by means of the method described in one of claims 1 to 13.

19. Use of the extract defined in one of claims 17 to 18 or of the product described in one of claims 14 to 16 as an antioxidant, anti-inflammatory, antimicrobial, cell anti-degenerative, anticancer and free radical sequestration agent in food, cosmetic product and pharmaceutical formulations.

20. Use of the extract defined in one of claims 17 to 18 or of the product defined in one of claims 14 to 16, as agent forming part of emulsions, nano and microencapsulations or adsorbed in other substances in order to improve their physico-chemical properties and/or stability.

21. Combined use of hydroxytyrosol and DHFG, or of HT and hydroxytyrosyl acetate in mixtures in hydrophilic and lipophilic media, which are or not forming emulsions in food, cosmetic products and pharmaceutical formulations.

## Amended claims

### Amended claims under Art. 19.1 PCT

1. Method for sequentially obtaining extracts with a high concentration of hydroxytyrosol, in a mixture of hydroxytyrosol and 3, 4-dihydroxyphenylglycol and in hydroxytyrosol acetate, **characterized in that** said method comprises carrying out at least one column chromatography of a hydroxytyrosol source, using a mixture of at least two ionic resins, preferably an anionic resin with a cationic resin.

2. Method according to claim 1, **characterized in that** the hydroxytyrosol source is solid or semisolid and one previous step a) is carried out, which comprises carrying out a liquid phase extraction and separation by filtration, centrifugation, decantation or a combination of centrifugation and decantation.

3. Method according to claim 1, **characterized in that** the hydroxytyrosol source is liquid and a previous step a) is carried out, which comprises carrying out a hydrolysis.

4. Method according to claim 1, **characterized in that** the hydroxytyrosol source is selected from alpechin, olive pomace, *alperujo,* leaves, pruning, bone, dressing process waters, oleaceae family plants and combinations thereof.

5. Method according to claim 1, **characterized in that** the anionic resin and cationic resin mixture contains at least 2% by weight of the total mixture in cationic resin.

6. Method according to claim 1, **characterized in that** a partial activation of the resin mixture is carried out at room temperature with a solution selected from an acid solution, an alkaline solution and a saline disolution, or with a solution selected from an acid solution, an alkaline solution and a saline dissolution, heating at temperatures of at least 50°C during regeneration.

7. Method according to claim 1, **characterized in that** the hydroxytyrosol source is passed through the column and said source is emptied before passing an eluent in order to carry out the chromatography.

8. Method according to claims 1 or 7, **characterized in that**, in order to carry out the column chromatography, the hydroxytyrosol source is loaded in the same place where the source is produced by mobile columns or in plant.

9. Method according to claim 1, **characterized in that** it comprises a hydroxytyrosol acetylation step after the elution of the column.

10. Method according to claims 1 or 9, **characterized in that** the column chromatography comprises a sequential discharge of said column with neutral or acidified water in a first series of elutions in a first discharge step, and with neutral or acidified water in a second series of elutions in a second discharge step.

11. Method according to claim 9 or 10, **characterized in that** when carrying out the column chromatography with a mixture of ionic resins, the sequential discharge of the column comprises a third step:
- emptying the column,
- subsequently completely flooding of the column with ethyl acetate, closing the inlet and outlet of the column and heating,
- and emptying the column once again.

12. Method according to claim 1 or 10, **characterized in that** an acetylation is carried out, which comprises:
- carrying out the column chromatography obtaining hydroxytyrosol extract or already purified hydroxytyrosol, putting a cationic resin in contact with said hydroxytyrosol extract or purified hydroxytyrosol and carrying out the acetylation reaction in a stirred tank, applying a temperature of above 50°C in the presence of ethyl acetate.

13. Method according to claim 1, **characterized in that** it also comprises a purification step for the extract obtained, which comprises carrying out one or more of the following operations:
- carrying out an adsorption chromatography with non-ionic resins and subsequently desorption,
- purification by means of adsorption on a polymeric phenolic fraction, said fraction retaining hydroxytyrosol or the extract formed by hydroxytyrosol and 3,4-dihydroxyphenylglycol, and redissolution of said hydroxytyrosol and 3,4-dihydroxyphenylglycol in a joint or individual manner,
- concentration of the extract or the purified compounds by means of evaporation, atomization or reverse osmosis, obtaining a dry or partially wet product.

14. A product selected from the compounds hydroxytyrosol, hydroxytyrosol acetate and 3, 4-dihydroxyphenylglycol, and combinations of said compounds, **characterized in that** said compound may be obtained by means of the method described in one of the claims 1 to 13.

15. A product which is a mixture of hydroxytyrosol and 3, 4-dihydroxyphenylglycol in a minimum ratio of 10:1 and a maximum ratio of 1:2, **characterized in that** it is obtainable by means of of the method described in one of claims 1 to 13.

16. A product according to claim 14, **characterized in that** it is selected from:
- hydroxytyrosol with a purity between 80-100%,
- a hydroxytyrosol and 3, 4-dihydroxyphenylglycol mixture with a purity between 60-100% as a sum of the individual ones,
- 3, 4-dihydroxyphenylglycol with a purity between 50-100%, and
- hydroxytyrosyl acetate with a purity between 80-100%.

17. A phenolic extract **characterized in that** it comprises at least one of the products defined in claims 14 to 16.

18. A phenolic extract **characterized in that** it is obtainable by means of the method described in one of claims 1 to 13.

19. Use of the extract defined in one of claims 17 to 18 or of the product described in one of claims 14 to 16 as an antioxidant, anti-inflammatory, antimicrobial, cell anti-degenerative, anticancer and free radical sequestration agent in food, cosmetic product and pharmaceutical formulations.

20. Use of the extract defined in one of claims 17 to 18 or of the product defined in one of claims 14 to 16, as agent forming part of emulsions, nano and microencapsulations or adsorbed in other substances in order to improve their physico-chemical properties and/or stability.

21. Use of the product defined in one of claims 14 to 16, which consists of a combination of hydroxytyrosol and DHFG, or a combination of HT and hydroxytyrosyl acetate in mixtures in hydrophilic and lipophilic media, which are or not forming emulsions in food, cosmetic products and pharmaceutical formulations.
